# EUROPEAN PATENT APPLICATION

(11) **EP 1 818 054 A1**
(43) Date of publication of application: **15.08.2007**
(21) Application number: 06002736.4
(22) Date of filing: 10.02.2006
(51) Int. Cl.: A61K 31/28, A61K 48/00, A61K 49/06, A61K 49/12

(54) **Use of a gadolinium chelate for labeling cells**

(71) Applicant: Deutsches Krebsforschungszentrum Stiftung des öffentlichen Rechts, 69120 Heidelberg (DE); Ruprecht-Karls-Universität Heidelberg, 69117 Heidelberg (DE); Klinikum Mannheim GmbH, 68167 Mannheim (DE)
(72) Inventor: Giesel, Frederik, 69120 Heidelberg (DE); Zechmann, Christian, 68167 Mannheim (DE); von Tengg-Koblingk, Hendrik, 69121 Heidelberg (DE); Essig, Marco, 69168 Wiesloch (DE); Tröster, Helmut, 68165 Mannheim (DE); Stroick, Mark, 68167 Mannheim (DE); Griebe, Martin, 68167 Mannheim (DE); Fatar, Marc, 67069 Ludwigshafen (DE)
(74) Representative: Schüssler, Andrea

(57) **Abstract**

Described is the use of a gadolinium chelate, preferably Gadofluorine M, for the preparation of a diagnostic composition for labeling cells, preferably stem cells, e.g., to render said cells detectable to magnetic resonance imaging and/or light microscopy.

## Description

The present invention relates to the use of a gadolinium chelate complex, preferably 6-N-[1,4,7-Tris(carboxylatomethyl)]-1,4,7,10-tetraazacyclododecan-10-N-(pentanoyl-3-aza-4-oxo-5-methyl-5-yl)]-2-N[1-O-alpha-D-carbonylmethylmanoopyranose]-L-lysin-[1-(4-perfluoroctylsulfonyl)-piperazin]-amid, Gd-complex (in the following called Gadofluorine M), for the preparation of a diagnostic composition for labeling cells, preferably stem cells, e.g., to render said cells detectable to magnetic resonance imaging or light microscopy.

Initially, Gadofluorine M was synthesized as a compound that could serve as a contrast enhancement agent for X-ray or Magnetic Resonance Imaging (MRI) (DE 100 40 381 C1). In its presence, imaging features of the sites where it was accumulated in an organism or in an organ change such that contrast differences to surrounding regions with lower Gadofluorine M concentrations can be used for the visualization of target structures. This feature is caused by the presence of a gadolinium (Gd) ion complexed with a chelator part of the molecule Gadofluorine M. Gd changes the way how surrounding water molecules behave in a strong magnetic field such that the imaging contrast in MRI is improved. In general, these contrast enhancers are administered to the cell by a process which resembles the so-called transfection principle. Transfection means to offer a mixture of the molecule of interest with an agent that is known to mediate an unspecific way of uptake of itself, complexed to the molecule of interest, into cells. In most of the cases, the molecule of interest is a nucleic acid molecule that enters the target cell nucleus in an unknown manner and is somehow integrated into the cellular genome, thus changing the genetic features of the cell. This uptake by the cells is variable with regard to its efficiency. Depending on the cell type used, the transfection efficiency is in the range of 0% to about 50%.

The therapeutic potential of administering stem cells, e.g., to promote tissue regeneration after myocardial infarction, stroke or Parkinson's disease has recently been demonstrated. So far, labelling of stem cells for monitoring cell therapy in vivo by MRI was carried out by loading the cells with small iron oxide particles (SPIO) or ultra small particles of iron oxide (USPIO). However, unfortunately, the incorporated particles generate a signal extinction in the magnetic field which is not restricted to the true anatomic region and, as a consequence, the degree of migration of the stem cells and their nidation, respectively, are overestimated. Moreover, blood deposition interferes with correct evaluation of the results. Thus, SPIO or USPIO labelled cells are not suitable for controlling stem cell therapy by MRI, in particular as regards animal models of cerebral haemorrhage and primary ischemic stroke, since in general haemorrhages occur in these pathologies and the labeled cells cannot be distinguished from extravascular blood.

In order to overcome these disadvantages the signal-positive labelling for contrasting the stem cells using known gadolinium chelates was studied, however with limited success. It could be shown in an in vitro model, that loading of cells and their use for signalling by MRI, in particular stem cells, can be achieved if the gadolinium chelates are introduced by use of transfection media like Lipofectin. However, the results obtained were not very promising, since the concentration of the contrast medium introduced by transfection into the cells was quite low resulting in a poor T1 weighted signalling and poor cell tracking, respectively.

The disadvantages of the current approaches for tracking cells, e.g., stem cells, by MRI can be summarized as follows:
- poor local delinitation within the tissue and, as a consequence, poor signal quantification;
- when using complexes of gadolinium chelates MRI requires the use of Lipofectin for transfection. However, this approach is not very efficient and it is highly questionable whether a uniform saturation of all cells can be achieved.

Therefore, it is the object of the present invention to provide a means which overcomes the disadvantages of the tools of the prior art for MRI, i.e. which allows to efficiently label cells with a label yielding good and reliable signal quantification, e.g., for cell tracking.

According to the present invention this is achieved by the subject matters defined in the claims.

During the experiments leading to the present invention administration of a mixture of Gadofluorine M with a transfection reagent called "Lipofectin" to the system of interest, e.g. human mesenchymal stem cells derived from lipoaspirates, could be proven to be successful as measured by MRI. Surprisingly, even better efficiency of Gadofluorine M uptake into the cells was found when Lipofectin was omitted. Thus, it was decided to have a closer look to this phenomenon and, for this purpose, a red fluorescent molecule (Cy 3.5) was covalently attached to Gadofluorine M. With such a fluorescent molecule the Gadofluorine M features could be studied much less time-consuming than with MRI, just by light microscopic investigation of living and fixed cells, i.e. performing phase contrast and corresponding fluorescence microscopy. MRI experiments showed that there is no difference between Gadofluorine M and Gadofluorine M with an additional fluorescent group attached thereto. Thus, a preferred embodiment of the present invention is a gadolinium chelate complex covalently coupled with a fluorescent dye, preferably Cy3-coupled Gadofluorine M. This embodiment has the advantage to be detectable via light/fluorescent microscopy (in view of the fluorescent dye)and MRI (in view of the Gd component).

The following features of the fluorescent Gadofluorine M were observed. Gadofluorine M i) was found first in the cells about 3 hours after application, ii) was linearly accumulating over more than one week within the cells apparently without any cell toxicity, iii) labeled 100% of the cells in a punctated pattern within the cytoplasm indicating a predominant accumulation of Gadofluorine M in cytoplasmic vesicles, iv) generated a labeling pattern similar to the PKH26 Fluorescent Cell Linker Kit dye which is known to integrate into biological membranes, v) was washed out of cells when fixed cells were brought into contact to Triton X-100 and ethanol, and vi) seemed not to negatively influence the growth, maintenance and capability to differentiate cells in cell culture over about 6 weeks.

Low efficiency of internalisation of MRI compounds is a general problem. Depending on the phagocytotic or endocytotic activity of the cells, the efficiency of uptake by the cells varies. Therefore, methods to mediate internalisation have been established. After testing several different gadolinium chelates the inventors focussed on one single MRI compound. There has been no case of such an efficient cellular uptake like that observed for Gadofluorine M yet. This fact and its obviously low toxicity makes it a tool for a variety of approaches.

A gadolinium chelate compound, preferably Gadofluorine M, is a highly efficient cell labelling compound with the potential to be used for long-term whole cell labelling in order to monitor the behaviour and the movements of labelled cells within an unlabelled cellular environment. For this purpose the features iii) and vi) are of particular interest. Since MRI- and light microscopic detectability are comprised in one molecule if the Gd-chelate compound is coupled to a fluorescent dye, the versatility of the compound allows parallel studies based on these features. The low toxicity of the compound makes it an ideal tool for long-term animal experiments focusing on long-lasting effects in a complex context. As the additional fluorescent compound was transported highly effective (100% of all cells), without loss of MRI signal intensity, this molecule has the potential to act as a carrier molecule to introduce molecules of interest into a cell. The data of the present study strongly suggest [(iii) and iv)] that one of the functional regions of Gadofluorine M acts like an anchor fixing the whole molecule to the cytoplasmic membrane from where these molecules are ingested into the cell body. This makes the molecule the ideal candidate to transport a molecule into the cell like a virus particle transports its genome into it. Since gene therapy applications are searching for efficient ways to direct genetic material into target cells, the Gadofluorine M^{™} molecule architecture offers a promising solution.

Thus, the present invention relates to the use of a gadolinium chelate, preferably Gadofluorine M, (a) for use in a diagnostic composition for labelling cells, preferably stem cells, preferably for rendering said cells detectable to MRI and/or light microscopy and (b) as a carrier molecule for the introduction of compounds of interest into cells.

Gadofluorine M has the following chemical formula:

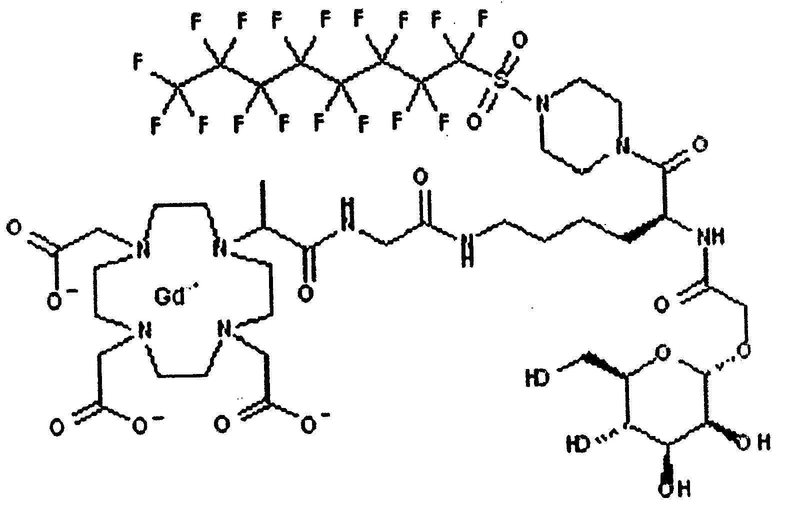

Examples of compounds of interest are drugs, prodrugs, proteins, genes, oligonucleotides, antisense-oligonucleotides, ribozymes, antibodies etc.

### Brief description of the drawings

Figure 1: Life microscopy from multiple parallel experiments that were carried out under absolutely identical conditions and finished after various periods
   For all images the same camera and the same exposure times and apertures were used, thus, allowing to make a direct comparison. The first fluorescent particles could be observed after 3 hours and fluorescence increased up to 48 hours.
Figure 2: MR-relaxometry values of control cells (left column) and various gadolinium chelates
Figure 3: 100% of the cells accumulate the contrast medium after an incubation time of 48 hours as shown by superposition of phase contrast and fluorescence images.
   Also shown are the original images prior to superposition.
Figure 4: Confocal-laser-scanning microscopy showing for the various images of the three colour channels Gadofluorine M in red, the lipid dye PKH2 in green and the nuclear dye DRAQ5 in blue
   The vesicular intracellular accumulation behaviour of the contrast medium as well as the similar distribution of the lipid dye can be seen. No separate series of images was prepared. Triton X as well as other solvents leach out the fluorescence labelled Gadofluorine M making it undetectable.
Figure 5: After six weeks, cell growth and morphology of the cells are unchanged
   The cells are still stably labelled. After labelling the cells are still capable of differentiating into adipogenic, chondrogenic and osteogenic cell lines. The capability to differentiate into various tissues is a substantial feature of the stem cells. This feature is not effected by labelling with Gadofluorine M.

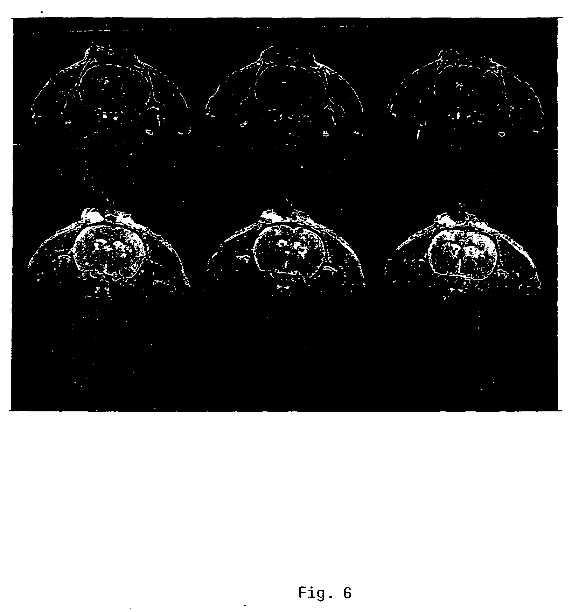

Figure 6: First in vivo assay of cells labelled with Gadofluorine M after stereotactic application of the cells (1 mio.) to a region which is opposite to a brain bleeding in a Highfield-MRI 9,4 T (Bruker)
   Cells of the upper image row (left) show a bright signal, the bleeding in the right part of the image looks less bright compared to the depiction in the T1 weighted MR-images. The lower image row shows T2 weighted MR-images with the bleeding (right part of the image) as well as the cells (left part of the image) are depicted darker which is due to susceptibility effects. This is confirmed by histological methods. Thus, this kind of labelling is capable of distinguishing labelled cells from effects of bleeding - which is in particular due to the combination of T1 and T2 weighted MRT images.

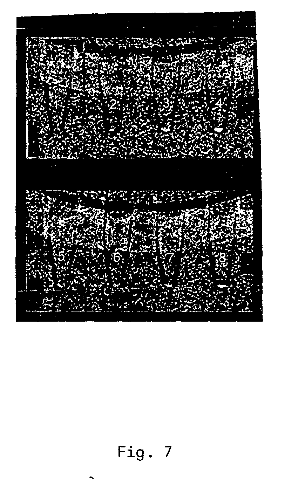

Figure 7: Comparison of various contrast agents
   1. Control cells (untreated)
   2. Cells + 10 µl Gadomer (500mmol Gd/ml)
   3. Cells + 10 µl Magnevist (500 mmol Gd/ml)
   4. Cells + 20 µl Gadofluorine M (250 mmol Gd/ml)
   5. Cells + 5 µl Gadovist (1000 mmol Gd/ml) + 100 µl Lipofectin
   6. Cells + 10 µl Gadomer (500 mmol Gd/ml) + 100 µl Lipofectin
   7. Cells + 10 ml Magnevist (500 mmol Gd/ml) + 100 µl Lipofectin
   8. Cells + 20 µl Gadofluorine M (250 mmol Gd/ml) + 100 µl Lipofectin

Introduction of gadolinium chelates in cells in comparison to other labeling methods was only one time experimentally performed with Magnevist (Daldrup-Link, Radiology 228(3) (2003), 760-767) as godalinium chelate using Lipofectin as mediating agent. Gadofluorine M was only used for lymphangiography in rabbits and was found to accumulate in degenerating peripheral nerves after i.v. application (Bendszus et al., Ann. Neurol. 57(3) (2005), 388-395; Misselwitz et al., Radiology 231(3) (2004), 682-688). As up to now gadolinium chelates were only used for extracellular applications after i.v. injection, no well known methods for introducing this chelate in cells existed. During the experiments leading to the present invention it was, however, found that introduction of the gadolinium chelate, e.g., Gadofluorine M, into the cells can be effected by the method described in example 1B. The synthesis of the gadolinium chelates can be carried out according to routine methods, e.g., the methods described in DE 100 40 381 C1.

After labeling of the cells according to standard methods and as described in the examples, such cells can then be exogenously applied to a host or a host tissue and monitored within the host using, e.g. MRI, or even light microscopy if the gadolinium chelate is coupled to e.g. a fluorescent dye. The labeled living cells of the present invention are of diagnostic, therapeutic or research value when introduced into a patient.

The term "cell" is understood to mean embryonic, fetal, paediatric, or adult cells or tissues, including but not limited to, stem cells, pluripotent stem cells, precursor cells, and progenitor cells. The term "cells" also encompasses virus particles and bacteria.

The term "host" means any mammalian patient or experimental subject, including human patients or subjects. The living cells of the current invention can be bone marrow cells, hematopoietic cells, tumor cells, lymphocytes, leukocytes, granulocytes, hepatocytes, monocytes, macrophages, fibroblasts, neural cells, mesenchymal stem cells, neural stem cells, and combinations thereof.

A further use of the gadolinium chelate compounds is as a carrier, i.e. transfection agent, for carrying compounds of interest, e.g. DNA, RNA or PNA, into cells.

The cells can be applied to the host to diagnose or cure a disease or to supply a cell type that is lacking or deficient in the host. The cells can also provide a drug or substance that is needed in the host for diagnostic, therapeutic, or experimental purposes.

In a preferred embodiment, the cells can be stem cells. Stem cells are cells that retain their ability to divide and to differentiate into specialized mature cells. Preferably, the cells are multipotent cells. In a particular preferred embodiment, the stem cells are gonadal stem cells, somatic stem/progenitor cells, haematopoietic stem cells, epidermal stem cells, mesenchymal stem cells or neuronal stem cells, i.e., multipotent cells from the nervous system which retain their ability to differentiate into oligodendrocytes. Oligodendrocytes are glial cells that myolinate or provide protective sheaths to neurons and axons in the central nervous system.

In the present invention the preferred cells are human cells with a special focus on non-embryonic human stem cells and non-human stem cells in particular.

The labelled cells can be applied for any diagnostic, therapeutic or experimental purposes. The cells, preferably stem cells, can be dispersed, or can be part of a tissue or organ or can be applied to any tissue or organ after the cells are labeled. The cells can be directly applied to the area to be treated or studied by means of surgery into the circulation or injection into a structure, organ, or body cavity in situ. When cells are integrated ex vivo into a tissue or organ, such tissue or organ can then be surgically applied or transplanted into a host. Preferably the cells are applied directly to a body structure.

The invention encompasses the use of MRI to monitor the movement, disposition and survival of the cells in the host. When cells are used that are immune cells, which react with a component of a disease process in the host, MRI monitoring can be used diagnostically to locate the cells attached to the disease process in the host. Immune cells are understood to encompass lymphoid or myeloid haematopoietic cells. Examples of such disease processes are malignant and metastasic diseases, degenerative diseases and infectious diseases. The cells can be used experimentally, in animal hosts, to study the development of diseases as a basis for designing therapeutic strategies. Tumor or neoplastic cells can be applied to animals as an experimental techniques to study the behaviour of neoplastic growth and metastasis in the organism. Moreover, the present invention encompasses not only gadolinium chelates like Gadofluorine M as a new substance for cell labelling which is detectable by MRI but also generally the therapeutic/diagnostic use of these chelates as a vehicle for the delivery of drugs, genes etc. into the cell nucleus.

Cells can be used to replace injured or diseased cells in the host, examples are diseases of the nervous system, injuries to the nervous system, injuries or diseases of bone, muscle, heart, circulation, internal organs, skin, interstitial tissue, mucosa, lungs, and gastrointestinal tract. When cells are used in this way, the host can be scanned with MRI to establish the location of the cell movement or migration of any of the cells, and the survival of the cells. The host can be scanned with MRI as frequently and during a period of time as required or desirable to monitor the cells.

The present invention also relates to a cell labeled with a compound as described above.

Further preferred uses are:

Use of the labeled cells for the preparation of a diagnostic composition allowing to track in vivo said cells;

Use of the labeled cells for the preparation of a diagnostic composition for monitoring the therapeutic effect of said cells. Preferably, said therapeutic effect is a long-term effect.

Preferably, tracking or monitoring of the labeled cells is performed by use of a Highfield MR Scanner (preferably > 1.5 Tesla) .

The following Example illustrates the invention.

### Example

### Results of labeling of cells/tissues with Gadofluorine M^{™} and visualization using various methods

### (A) Mesenchymal stem cells (MSC) preparation and characterization

Cells were processed and characterized as described previously (Zuk et al., Tissue Eng. 7(2) (2001), 211-228). Before being used in the animal experiments, the cells were tested for their multilineage differentiation potential. The cells were directed towards the osteogenic, chondrogenic and adipogenic lineage using differentiation kits (Poietcs TM, Cambrex Bio Science, Verviers, Belgium). For further differentiation of the MSC, surface protein expression was analyzed by flow cytometry, showing positive staining for the typical MSC-markers CD44, CD73, CD29 and CD90 and negative findings for markers of the haematopoetic lineage CD14, CD34, CD45, the stem cell marker CD133, or the marker for endothelial cells CD144 (Kern et al.,Comparative Analysis of Mesenchymal Stem Cells from Bone Marrow, Umbilical Cord Blood or Adipose Tissue, Stem Cells, 2006) .

### (B) Cell labeling with various contrast agents

According to Daldrup-Link et al. (Radiology 228 (3) (2003), 760-7) a modified protocol with and without Lipofectin (Invitrogen, Karlsruhe, Germany, #18292-011) as a mediator for the gadolinium uptake was used. Transfection with Lipofectin was achieved as follows: 100 µl Lipofectin were dissoloved in 100 µl DMEM. In separated tubes Gadofluorine M, Magnevist^{™}, Gadovist^{™} or Gadomer-17 (all from Schering AG, Berlin, Germany) were dissolved in 100 µl DMEM. After that, the tubes were mixed and filled up with DMEM up to a final volume of 1 ml and incubated at 37°C/5% CO₂ for 45 minutes. Before adding these contrast agent solutions to the cells, the cells were washed with PBS and regular MSC growth medium without antibiotics, according to the recommendations of the manufacturer of Lipofectin, was added to the cells.

### Cell-labeling without Lipofectin was achieved as follows:

Gadofluorine M, Magnevist^{™}, Gadovist^{™} or Gadomer-17 were dissolved in 1 ml DMEM and incubated at 37°C/5% CO₂ for 45 minutes. Before adding these contrast agent solutions to the cells, the cells were washed with PBS and regular MSC growth medium without antibiotics, according to the recommendations of the manufacturer of Lipofectin, was added to the cells.

### (C) Comparison of the different contrast agents by MRI

After incubation for 24 h the cell fractions of the supernatants were frozen for a later analysis and cells were washed 2 times with PBS, detached with trypsin and after a first centrifugation resuspended in 1 ml complete MSCGM growth medium (Cambrex Bioscience, Verviers, Belgium, #PT-4106). After that, cells were again centrifuged to form a compact pellet for the MRI scan. The following conditions were examined 3 times to establish stable results for a later quantitative analysis of the MRI signals: MSC without gadolinium; only incubated with normal MSCGM (negative control), MSC with Lipofectin, MSC with Lipofectin and the various contrast agents (Gadofluorine M, Magnevist^{™}, Gadovist^{™}, Gadomer-17) and MSC without Lipofectin and the various contrast agents.

MR imaging was performed on a 1.5 T clinical MR scanner using a dedicated coil and a T1-weighted sequence. For the MRI examination the probes were placed in a water bath at 37°C. MR sequences:
1. Localizer
   Samples in T1 contrast
2. T1-weighted spin-echo-sequence: i. SL=1,11; DS=3,0 mm; ST=3,0 mm; PS=0,21 x 0,21 mm. ii. FA=90; TR=540 ms; TE=12 ms.
3. Relaxation measurements (T1-w with different TI): i. T1=100, 300, 500, 700, 1000, 1500, 2000, 2760.

### (D) Results

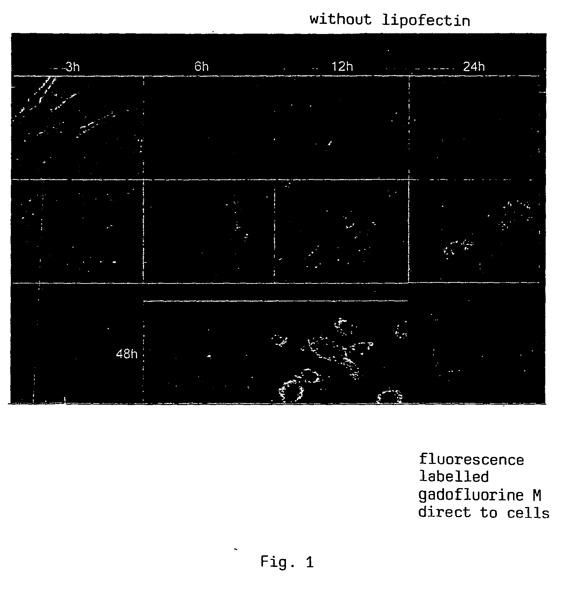

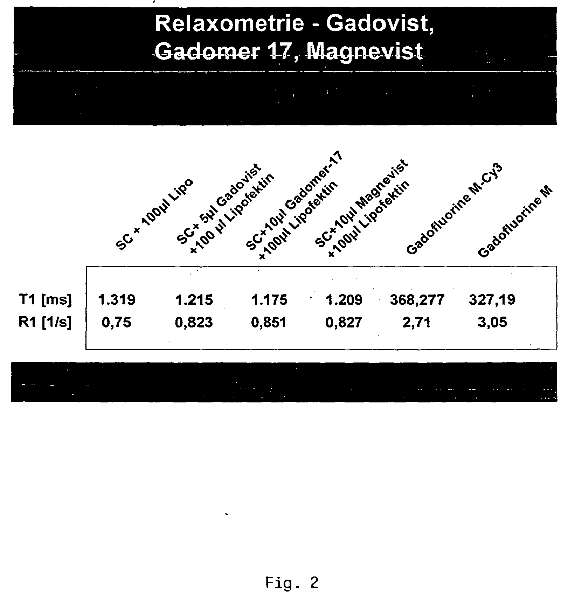

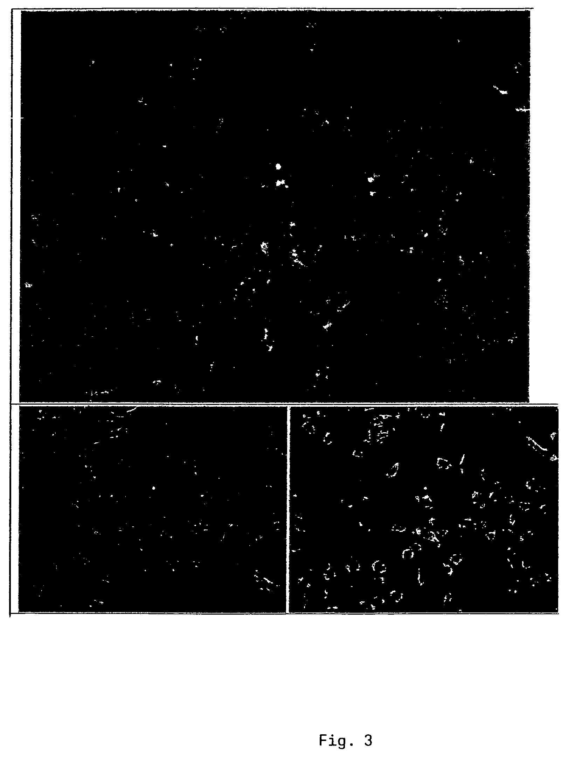

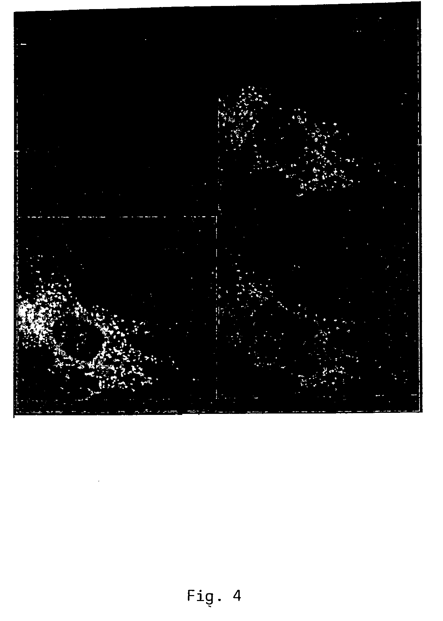

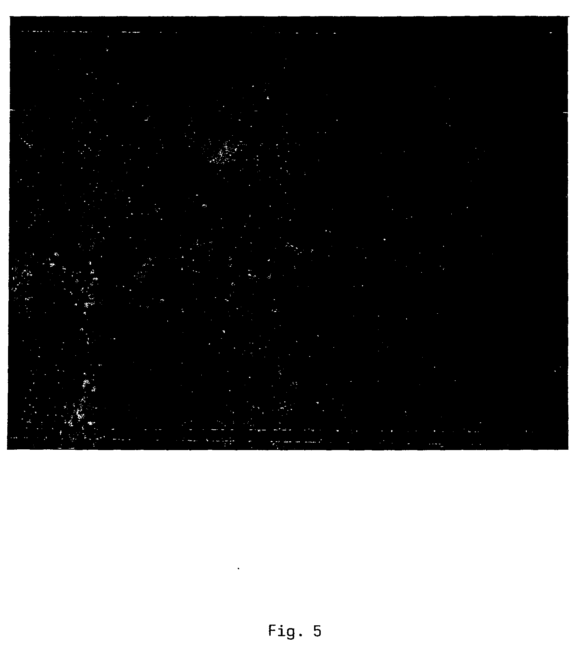

The results are shown in Figures 1 to 7; see figure legends for further information.

## Claims

1. Use of a gadolinium chelate compound for the preparation of a diagnostic composition for labeling a cell or as a carrier molecule for the introduction of compounds of interest into cells.

2. Use according to claim 1, wherein the gadolinium chelate compound is 6-N-[1,4,7-Tris(carboxylatomethyl)]-1,4,7,10-tetraazacyclododecan-10-N-(pentanoyl-3-aza-4-oxo-5-methyl-5-yl)]-2-N[1-O-alpha-D-carbonylmethylmanoopyranose]-L-lysin-[1-(4-perfluoroctylsulfonyl)-piperazin]-amid, Gd-complex (Gadofluorine M):

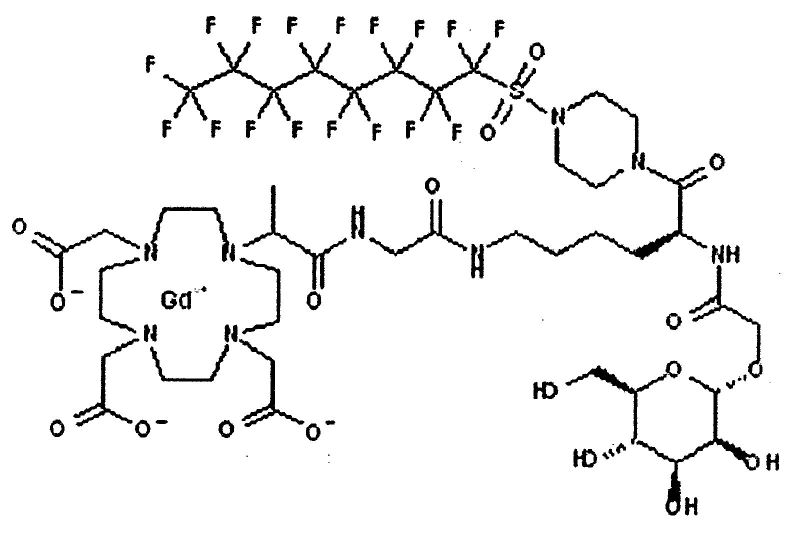

3. Use according to claim 1 or 2 wherein the gadolinium chelate compound is covalently attached to a fluorescent dye.

4. The use according to claim 1, 2 or 3 for labeling a cell to render said cell detectable to magnetic resonance imaging and/or light microscopy.

5. The use according to any one of claims 1 to 4, wherein said cell is a stem cell.

6. The use according to claim 5, wherein said stem cell is a gonadal stem cell, somatic stem/progenitor cell, haematopoietic stem cell, epidermal stem cell, mesenchymal stem cell or neuronal stem cell.

7. The use according to any one of claims 1 to 6, wherein said cell is a human cell.

8. Cell as defined in any one of claims 1 to 7 which is labeled with a complex as defined in any one of claims 1 to 7.

9. Use of the cell of claim 8 for the preparation of a diagnostic composition for tracking in vivo said cell.

10. Use of the cell of claim 8 for the preparation of a diagnostic composition for monitoring the therapeutic effect of said cell.

11. The use according to claim 10, wherein said therapeutic effect is a long-term effect.

12. The use according to any one of claims 9 to 11, wherein tracking or monitoring is performed by use of a highfield MR Scanner.
